Europäisches Patentamt

European Patent Office

Office européen des brevets

(11) Publication number: **0 348 560**
A1

# EUROPEAN PATENT APPLICATION

(21) Application number: 88305820.8

(51) Int. Cl.⁴: **A61K 7/16**

(22) Date of filing: 27.06.88

(43) Date of publication of application:
03.01.90 Bulletin 90/01

(84) Designated Contracting States:
AT BE CH DE ES FR GB IT LI NL SE

(71) Applicant: LION CORPORATION
3-7, Honjo 1-chome
Sumida-ku Tokyo(JP)

(72) Inventor: Yamazaki, Yoji
No. 440-7, Tokunobu
Hiratsuka-shi Kanagawa-ken(JP)

(74) Representative: Stuart, Ian Alexander et al
MEWBURN ELLIS & CO. 2/3 Cursitor Street
London EC4A 1BQ(GB)

(54) Oral composition.

(57) There is provided an oral composition comprising α-bisabolol and menthol, eugenol or 3-octanol. The oral composition suppresses the formation of dental plaque in the oral cavity and is effective in preventing dental caries and periodontal disease. A preferred composition has, by weight, 0.0001-5% of α-bisabolol and 0.001-5% of menthol, eugenol, 3-octanol or a mixture.

EP 0 348 560 A1

Xerox Copy Centre

## ORAL COMPOSITION

The present invention relates to an oral composition which suppresses the formation of dental plaque in the oral cavity and is effective in preventing dental caries and also in preventing periodontal desease.

Dental plaque firmly adhering to the surface of teeth is composed of about 70% bacteria, about 20% polysaccharides produced by the bacteria and about 10% food remains. It is said that acids stored in dental plaque decalcify enamel, causing dental caries, and in addition, bacteria and toxins produced in dental plaque cause gingivitis and periodontitis. Therefore, dental plaque is observed as a cause of dental caries and periodontal disease which are regarded as two main oral diseases.

Formation of dental plaque is accelerated due to the synthesis of polysaccharides from sucrose by oral bacteria, especially Streptococcus mutans. In more detail, Streptococcus mutans synthesizes adhesive polysaccharides such as dextran and mutan from sucrose through the production of GTF (glucosyltransferase, dextran-synthesizing enzyme). The thus synthesized polysaccharides incorporate Streptococcus mutans as well as other bacteria, forming dental plaque having a given bacterial flora. In addition, bacteria such as Streptococcus mutans produce acids by utilizing various kinds of sugar and the thus produced acids decalcify the surface of enamel by remaining in dental plaque. On the other hand, dead bacteria and various substances or toxins produced in dental plaque give a bad influence upon gingivae, resulting in periodontal disease.

Accordingly, it is desirable to suppress the formation of dental plaque in order to prevent dental caries and periodontal disease.

The present assignee has proposed an oral composition comprising amygdalin, indigo, sanshool, bisabolol or rutin as an effective ingredient which can suppress the formation of dental plaque due to Streptococcus mutans (Japanese Patent Application Laid-open No. 58- 213706). The oral composition is effectively used in preventing dental caries and periodontal disease.

However, there is a need for an oral composition which can suppress the formation of dental plaque more effectively.

## SUMMARY OF THE INVENTION

It is an object of the invention to provide an α-bisabolol-containing oral composition which can prevent dental caries and periodontal disease more effectively.

The present inventors carried out a series of researches on increasing the effect on suppressing the formation of dental plaque by α-bisabolol. As the result, it was found that, when α-bisabolol is combined with menthol, eugenol or 3-octanol, the formation of dental plaque due to Streptococcus mutans is remarkably suppressed because of a synergistic effect based on an interaction between α-bisabolol and menthol, eugenol or 3-octanol, resulting in effective prevention of dental caries and periodontal disease.

More detailedly, if menthol, eugenol, 3- octanol or α-bisabolol is singly used in a low concentration, respectively, each of them exerts little effect on suppressing the formation of dental plaque. Nevertheless, when α-bisabolol is combined with menthol, eugenol or 3-octanol, an extremely high effect on suppressing the formation of dental plaque due to Streptococcus mutans is attained even if the compounds are used in a lower concentration. Further, the synergistic effect is brought about only by the combination of α-bisabolol and menthol, eugenol or 3-octanol. Even though the other flavors such as methyl salicylate, carvone or cineol are combined with α-bisabolol, such a synergistic effect is no longer attained.

Accordingly, the present invention provides an oral composition which can suppress the formation of dental plaque comprising α-bisabolol and a compound selected from the group consisting of menthol, eugenol and 3-octanol and mixtures thereof.

The above and other objects, features and advantages of the present invention are more apparent from the following description.

## DETAILED DESCRIPTION OF THE INVENTION

The oral composition of this invention is available in various forms applicable to the mouth such as dentifrices (including toothpaste, toothpowder and liquid dentifrice), mouthwashes, dental pastes, gingiva massage creams, troches, gargle tables, chewing gums, or the like.

The oral composition contains α-bisabolol. α-Bisabolol includes isomers, i.e. α-(-)-bisabolol, α-(+)-bisabolol and α-(±)-bisabolol which is a mixture of α-(-)-bisabolol and α-(+)-bisabolol.

The blending amount of α-bisabolol may be 0.0001 to 5 % by weight, preferably 0.001 to 2% by weight of the composition.

The oral composition also contains a compound selected from the group consisting of menthol, eugenol and 3-octanol, and mixture thereof. The blending amount of the compound may be 0.001 to 5% by weight, preferably 0.005 to 2% by weight of the composition.

The oral composition of this invention may be incorporated further with other commonly used ingredients which are selected according to the type of preparations.

For dentifrice, it may be incorporated with abrasive such as calcium secondary phosphate dihydrate, calcium secondary phosphate anhydride, calcium primary phosphate, calcium tertiary phosphate, calcium carbonate, calcium pyrophosphate, insoluble sodium metaphosphate, amorphous silica, crystalline silica, aluminosilicate, aluminum silicate, aluminum oxide, aluminum hydroxide, magnesium tertiary phosphate, magnesium carbonate, magnesium sulfate, titanium oxide, and resin.

For toothpaste, it may be incorporated with a binder such as sodium carboxymethylcellulose, methylcellulose, sodium carboxymethylhydroxyethylcellulose, hydroxyethylcellulose, sodium alginate, carrageenan, gum arabic, xanthane gum, tragacanth gum, calaya gum, polyvinyl alcohol, sodium polyacrylate, carboxyvinyl polymer, and polyvinyl pyrrolidone, and a humectant such as polyethylene glycol, ethylene glycol, sorbitol, glycerin, propylene glycol, 1,3-butylene glycol, xylit, maltit, and lactit.

The oral composition of this invention may be incorporated further with one kind or more of anionic, nonionic, cationic, and amphoteric surface active agents. Example of the anionic surface active agents include sodium lauryl sulfate, sodium myristyl sulfate, and other water-soluble salts of alkyl sulfates having an alkyl group of 8 to 18 carbon atoms; sodium coconutmonoglyceride sulfonate and other water-soluble higher acid monoglyceride sulfonates derived from a higher fatty acid having 10 to 18 carbon atoms; sodium salts of α-olefin sulfonate, paraffin sulfonate, and N-methyl-N-palmitoyltauride; sodium N-lauroyl sarcosinate; and sodium N-lauroyl-β-alanine. Examples of the nonionic surface active agents include lauroyl diethanolamide and other fatty acid alkanolamides; sucrose monolaurate, sucrose dilaurate, and other sucrose fatty acid esters; polyoxyethylene sorbitan fatty acid ester, polyoxyethylene fatty acid ester, polyoxyethylene hardened castor oil derivative, lactose fatty acid ester, lactitol fatty acid ester, maltitol fatty acid ester, and polyoxyethylene-polyoxypropylene block copolymer.

The oral composition of this invention may be incorporated further with a sweetener such as saccharin sodium, stevioside, neohesperidyldihydrochalcone, glycyrrhizin, perillartine, and p-methoxycinnamic aldehyde, and a preservative.

In preparation of dentifrice, the blending amount of abrasive is preferably in the range of 1 - 99%, particularly 10 - 70% by weight of the composition. The blending amount of binder is preferably in the range of 0 - 5%, particularly 0.1 - 5% by weight of the composition. The blending amount of humectant is preferably in the range of 10 - 80%, particularly 30 - 60% by weight of the composition. The blending amount of surface active agent is preferably in the range of 0.1 - 5%, particularly 0.5 - 2% of the composition. The blending amount of sweetener is preferably 0.01 - 5%, particularly 0.05 - 2% by weight of the composition. The blending amount of flavor is preferably in the range of 0.1 - 5%, particularly 0.5 - 2% by weight of the composition.

The pH of the oral composition in liquid or paste type may preferably be in the range of 5 -10.

The oral composition may further contain other effective ingredients in an effective amount including dextranase, protease, lysozyme, lytic enzyme, mutanase, sorbic acid, alexidine, β-glycyrrhetinic acid, hinokitiol, chlorhexidine, alkylglycine, alkyldiaminoethylglycine hydrochloride, allantoin, ε-aminocaproic acid, tranexamic acid, sodium monofluorophosphate, sodium fluoride, stannous fluoride, azuren, vitamin E, water-soluble primary and secondary phosphate, cetylpyridinium chloride and other quaternary ammonium compounds, sodium chloride, crude drug extracts.

For other types of formulation than mentioned above, proper ingredients can be incorporated in the usual way.

The invention is now described with reference to the following examples, although the invention is not limited to the examples. "Percent (%)" in the formulations of the examples denotes "% by weight".

EXAMPLE 1

α-Bisabolol, menthol, eugenol, 3-octanol, methyl salicylate, carvone and cineol are used as samples to evaluate their effects on inhibiting the formation of dental plaque by Incubation Adhesion Method.

Incubation Adhesion Method

A predetermined amount (0, 4 or 8 μg/mℓ) of α-bisabolol and 125 μg/mℓ of menthol, eugenol, 3-octanol, methyl salicylate, carvone or cineol were added to a BHI (brain heart infusion) medium containing 1% of sucrose. Then, Streptococcus mutans (strain 6715) was inoculated to the medium and incubated at 37°C for 16 hours under an anaerobic condition ($N_2$: $CO_2$ :$H_2$ = 80:10:10). After incubation, the resulting dental plaque was washed twice with water and dispersed in the same amount of 0.5-N NaOH solution. To determine the amount of dental plaque formed, the suspension of dental plaque was examined for absorbance (turbidity) at a wavelength of 550 nm.

The results are shown in Table 1.

Inhiviting rate of dental plaque formation (%)

$$= \frac{A - B}{A} \times 100$$

A : Absorbance (O.D. 550nm) of the control
B : Absorbance (O.D. 550nm) of the sample

Synergistic effect

◎ : Inhibiting rate is not less than 80%
○ : Inhibiting rate is not less than 50%, less than 80 %
△ : Inhibiting rate is not less than 20%, less than 50 %
× : Inhibiting rate is less than 20%

Table 1

| Content of α-bisabolol (μg/mℓ) | Sample | Amount of dental plaque formation (O.D., 550nm) | Inhibiting rate of dental plaque formation (%) | Synergistic effect |
|---|---|---|---|---|
| 0 | Control | 0.62 | 0 | - |
| | Menthol | 0.60 | 3.2 | - |
| | Eugenol | 0.62 | 0 | - |
| | 3-Octanol | 0.59 | 4.8 | - |
| | Methyl salicylate | 0.61 | 1.6 | - |
| | Carvone | 0.62 | 0 | - |
| | Cineol | 0.62 | 0 | - |
| 4 | Control | 0.61 | 1.6 | - |
| | Menthol | 0.01 | 98.4 | ◎ |
| | Eugenol | 0.60 | 3.2 | × |
| | 3-Octanol | 0.58 | 6.5 | × |
| | Methyl salicylate | 0.61 | 1.6 | × |
| | Carvone | 0.60 | 3.2 | × |
| | Cineol | 0.59 | 4.8 | × |
| 8 | Control | 0.60 | 3.2 | - |
| | Menthol | 0.01 | 98.4 | ◎ |
| | Eugenol | 0.02 | 96.8 | ◎ |
| | 3-Octanol | 0.06 | 90.3 | ◎ |
| | Methyl salicylate | 0.39 | 37.1 | Δ |
| | Carvone | 0.42 | 32.3 | Δ |
| | Cineol | 0.53 | 14.5 | × |

As seen from the results of Table 1, the formation of dental plaque is synergistically suppressed by combining α-bisabolol with menthol, eugenol or 3-octanol.


EXAMPLE 2


| Toothpaste: | |
|---|---|
| Aluminum hydroxide | 45.0 % |
| Gelling silica | 2.0 |
| Sorbit | 25.0 |
| Sodium carboxymethylcellulose | 1.0 |
| Sucrose monopalmitate | 1.0 |
| Sodium lauryl sulfate | 1.5 |
| Sodium saccharin | 0.2 |
| Ethanol | 0.1 |
| Sodium benzoate | 0.1 |
| α-(-)-Bisabolol | 0.1 |
| Menthol | 0.2 |
| Flavor | 1.0 |
| Water | Balance |
| | 100.0 % |


EXAMPLE 3

| Toothpaste: | |
|---|---|
| Precipitated silica | 25.0 % |
| Sorbit | 25.0 |
| Glycerin | 25.0 |
| Polyvinyl pyrrolidone | 1.0 |
| Lauroyl polyglycerin ester | 1.0 |
| Polyoxyethylene (60 mol) sorbitan monolaurate | 0.5 |
| Sodium saccharin | 0.2 |
| Ethyl parahydroxybenzoate | 0.1 |
| Chlorhexidine hydrochloride | 0.1 |
| $\alpha$-(+)-Bisabolol | 0.03 |
| Menthol | 0.2 |
| Eugenol | 0.1 |
| Flavor | 1.0 |
| Water | Balance |
| | 100.0 % |

EXAMPLE 4

| Toothpaste: | |
|---|---|
| Calcium secondary phosphate dihydrate | 20.0 % |
| Calcium secondary phosphate anhydrade | 20.0 |
| Gelling silica | 2.0 |
| Sorbit | 20.0 |
| Propylene glycol | 2.5 |
| Sodium carboxymethylcellulose | 1.0 |
| Lauroyl diethanolamide | 1.0 |
| Sodium lauryl sulfate | 1.5 |
| Sodium lauroyl sarcosinate | 0.3 |
| Sodium saccharin | 0.1 |
| Ethyl parahydroxybenzoate | 0.1 |
| Tranexamic acid | 0.05 |
| $\alpha$-($\pm$)-Bisabolol | 0.05 |
| Menthol | 0.1 |
| 3-Octanol | 0.05 |
| Flavor | 0.8 |
| Water | Balance |
| | 100.0 % |

EXAMPLE 5

6

| Oral paste: | |
| --- | --- |
| Cetanol | 10.0 % |
| Squalane | 20.0 |
| Abrasive precipitated silica | 5.0 |
| Polyoxyethylene (40 mol) hardened castor oil | 0.1 |
| Sorbitan monooleate | 1.0 |
| Glycyrrhetinic acid | 0.1 |
| Sodium saccharin | 0.6 |
| Flavor | 0.6 |
| $\alpha$-(-)-Bisabolol | 0.03 |
| Eugenol | 0.2 |
| Water | Balance |
| | 100.0 % |

EXAMPLE 6

| Oral paste: | |
| --- | --- |
| Liquid paraffin | 15.0 % |
| Cetanol | 10.0 |
| Glycerin | 20.0 |
| Sorbitan monopalmitate | 0.6 |
| Polyoxyethyle (40 mol) sorbitan monostearate | 5.0 |
| Chlorhexidine gluconate | 0.1 |
| Flavor | 0.5 |
| $\alpha$-($\pm$)-Bisabolol | 0.2 |
| Menthol | 0.2 |
| Water | Balance |
| | 100.0% |

EXAMPLE 7

| Mouthwash: | |
| --- | --- |
| Sorbit | 10.0 % |
| Ethanol | 5.0 |
| Polyoxyethyle (60 mol) hardened castor oil | 0.1 |
| Sucrose monopalmitate | 0.2 |
| Sodium saccharin | 0.2 |
| Flavor | 0.6 |
| $\alpha$-($\pm$)-Bisabolol | 0.1 |
| Eugenol | 0.1 |
| Menthol | 0.1 |
| Water | Balance |
| | 100.0 % |

EXAMPLE 8

7

| Oral troche: | |
|---|---|
| Lactose | 99.0 % |
| Chlorhexidine gluconate | 0.05 |
| Polyoxyethyle (60 mol) monostearate | 0.2 |
| α-(-)-Bisabolol | 0.02 |
| Menthol | 0.2 |
| 3-Octanol | 0.05 |
| Stevia extract | 0.2 |
| Flavor | 0.01 |
| Hydroxyethyl cellulose | Balance |
| | 100.0 % |

## Claims

1. An oral composition which can suppress the formation of dental plaque comprising α-bisabolol and a compound selected from menthol, eugenol and 3-octanol, and mixtures of two or more thereof.

2. The oral composition according to claim 1, wherein α-bisabolol is blended in an amount of 0.0001 to 5% by weight of the composition and the compound selected from the group consisting of menthol, eugenol and 3-octanol, and mixture thereof is blended in an amount of 0.001 to 5% by weight of the composition.

3. The oral composition according to claim 1, wherein the composition is a dentifrice.

4. A composition comprising (i) α-bisabolol and (ii) a compound selected from menthol, eugenol and 3-octanol, and mixtures of two or more thereof as a combined preparation for simultaneous, separate or sequential use in suppressing the formation of dental plaque.

5. A composition according to any preceding claim including one or more orally acceptable materials selected from adjuvants, abrasives, binders, surface active agents, sweeteners, and orally effective ingredients.

6. Use of (i) α-bisabolol and (ii) a compound selected from menthol, eugenol and 3-octanol, and mixtures of two or more thereof in preparing a composition according to any preceding claim.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-1 081 170 (H. JANISTYN) <br> * Column 1 * <br> --- | 1-6 | A 61 K 7/16 |
| X | FR-A-2 272 670 (LUDWIG MERCKLE KG CHEM. PHARM. FABRIK) <br> * Page 3, line 37 - page 4, line 10; examples 2,4 * <br> --- | 1,2,4-6 | |
| Y,D | CHEMICAL ABSTRACTS, vol. 100, no. 16, 16th April 1984, page 348, abstract no. 126742v, Columbus, Ohio, US; & JP-A-58 213 706 (LION CORP.) 12-12-1983 <br> * Abstract * <br> --- | 1-6 | |
| Y | CHEMICAL ABSTRACTS, vol. 100, no. 25, 18th June 1984, page 67, abstract no. 203605g, Columbus, Ohio, US; & JP-A-59 29 619 (T. HASEGAWA CO., LTD) 16-02-1984 <br> * Abstract * <br> --- | 1-6 | |
| P,A | PATENT ABSTRACTS OF JAPAN, vol. 12, no. 242 (C-510)[3089], 8th July 1988; & JP-A-63 33 326 (KAO CORP.) 13-02-1988 <br> * Abstract * <br> --- | 1-6 | TECHNICAL FIELDS SEARCHED (Int. Cl.4) <br><br> A 61 K |
| A | DE-A-2 445 676 (GALENIKA Dr. HETTERICH GmbH) <br> * Page 1, line 1 - page 3, paragraph 2 * <br> --- | 1-6 | |
| A | GB-A-1 161 018 (COLGATE-PALMOLIVE CO.) <br> * Page 1, lines 32-40; page 3, lines 39-41; table 1 * <br> ----- | 1-6 | |

The present search report has been drawn up for all claims

| Place of search | Date of completion of the search | Examiner |
|---|---|---|
| THE HAGUE | 23-02-1989 | MUELLNERS W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)